Europäisches Patentamt

European Patent Office

Office européen des brevets

(19)

(11) Publication number: 0 036 715
A1

# (12) EUROPEAN PATENT APPLICATION

(21) Application number: 81300860.4

(22) Date of filing: 02.03.81

(51) Int. Cl.³: B 01 J 35/10

(30) Priority: 19.03.80 JP 34040/80

(43) Date of publication of application: 30.09.81
Bulletin 81/39

(84) Designated Contracting States: BE DE FR GB IT NL

(71) Applicant: TOA NENRYO KOGYO K.K.,
1-1 Hitotsubashi, 1-Chome Chiyoda-Ku, Tokyo (JP)

(72) Inventor: Kamiyama, Setsuo, 119-7, Ooaza Yamada,
Kawagoc-shi Saitama-ken (JP)
Inventor: Shiozawa Kouji, 1515, Ooaza Ishizaka,
Hatoyama-mura Saitama-Ken (JP)
Inventor: Nishikawa, Eiichiro, 1902-5, Ooaza Kamekubo
Ooi-machi, Iruma-gun Saitama-ken (JP)
Inventor: Kaneko, Katsumi, 2174-19, Ooaza
Nishitsuru-gaoka Ooi-machi, Iruma-gun Saitama-ken
(JP)

(74) Representative: Northover, Robert Frank et al, Esso
Chemical Research Centre P.O. Box 1, Abingdon
Oxfordshire, OX13 6BB (GB)

(54) Catalyst for diacyloxylation of conjugated dienes.

(57) A catalyst for the diacyloxylation of conjugated dienes such as butadiene, by reaction with a carboxylic acid such as acetic acid and oxygen, including a noble metal of Group VIII (preferably palladium) and at least one element from Group IV, V, or VI (preferably tellurium) on a silica carrier, the silica having a surface area of at least 200 m²/g and an average pore diameter of at least 100 Å which increases the catalyst life.

## CATALYST FOR DIACYLOXYLATION OF CONJUGATED DIENES

This invention relates to a catalyst for diacyloxylation of conjugated dienes, and more particularly to a solid catalyst for use in the manufacture of diacyloxyalkenes by reacting conjugated dienes, carboxylic acids and oxygen. The catalyst includes a carrier supporting at least one noble metal of Group VIII and at least one Group IV, V or VI element, excluding those in the second period.

Diacyloxyalkenes are useful as raw materials for a variety of industrial applications. For example, 1,4-butane-diol derived from 1,4-diacyloxybutene-2 is an industrially useful compound serving as a solvent, or an intermediate for the manufacture of tetrahydrofuran, γ-butyrolactone, polyesters, or the like.

It is known to manufacture diacyloxyalkenes by reacting a conjugated diene, a carboxylic acid and oxygen in the presence of a palladium catalyst, and various methods have hitherto been proposed for that purpose. For example, Japanese Patent Application Laid-Open Specification No. 72090/1973 discloses a method which employs a solid catalyst containing at least one metal selected from among palladium, tellurium and selenium, and No. 96513/1973 discloses a method which employs a solid catalyst containing at least one metal selected from among palladium, antimony and bismuth, and at least one selected from between tellurium and selenium. In order to improve the activity of these solid catalysts, it has been proposed to prepare such a solid catalyst be using an activated carbon carrier treated with nitric acid as disclosed in Japanese Patent Application Laid-Open Specification No. 11812/1974. For the same purpose, it has also been proposed to use a solid catalyst after reducing it, treating it with a gas containing molecular oxygen at a temperature of at least 200°C., and reducing it again, as disclosed in Japanese Patent Publication No. 12686/1977. There has also been developed a method employing a solid catalyst composed of three catalytic components, i.e., palladium, tellurium and at least one metal selected from among tin, germanium and lead, and having a high catalytic activity, as disclosed in U.S. Patent No. 4,225,727 granted September 30, 1980.

The aforementioned methods employing solid catalysts have the drawback that they undergo a marked reduction of catalytic activity with the lapse of time, although they show high activity and selectivity during an initial period of use. In order to prevent such reduction of activity with the lapse of time, it has been proposed to control the quantity of vinylcyclohexene in butadiene, formic acid in acetic acid, or like impurities, and incorporate a polymerization inhibitor into a reaction system for the manufacture of diacyloxyalkenes in the presence of a known solid catalyst (Japanese Patent Publication No. 15491/1978), and incorporate elemental sulfur as a degradation inhibitor (Japanese Patent Application Laid-Open Specification No. 51313/1977). Although these proposals have considerably improved the solid catalysts known in the art, all of the known catalysts still have a half life of activity which is not longer than 1,000 hours, and are difficult to apply for satisfactory use on an industrial basis.

It has also been proposed, as disclosed in Japanese Patent Application Laid-Open Specification No. 103509/1973, to provide a catalyst maintaining a high activity by using - instead of an activated carbon carrier - alumina, silica, silica-alumina or titania in which pores having a diameter of at least 1,000 Å occupy at least 80% of the total pore volume. No such carrier is, however, suitable for practical use, since any catalyst prepared by using it has a by far lower activity than any prior catalyst having an activated carbon carrier.

Applicants have now found that a catalyst prepared by using, as a carrier, silica having a specific pore structure can maintain a high activity for an extremely long period of time.

This invention thus provides an improvement in the catalyst for diacyloxylation of conjugated dienes, the catalyst including a carrier supporting thereon at least one noble metal belonging to Group VIII of the periodic table, and at least one element belonging to Groups IV, V or VI, particularly Groups IVA, VA and VIA of the Fisher Scientific Company periodic table, (but excluding those of the second period), wherein the carrier comprises silica having a specific surface area of at least 200 $m^2/g$, and an average pore diameter of at least 100 A.

The use of a silica having a specific surface area of less than 200 $m^2/g$ results in a catalyst having a low activity, even if it has an average pore diameter of at least 100 A. Likewise, the use of any silica having an average pore diameter which is smaller than 100 A results in a catalyst having a low level of activity which undergoes a sharp reduction with the lapse of time, even if it has a specific surface area of at least 200 $m^2/g$. It is thus imperative to use silica which satisfies both of the requirements. Preferably, the catalyst of this invention is prepared by using silica having a specific surface area of 250 to 1,000 $m^2/g$, and an average pore diameter of 100 to 500 A.

For the purpose of this invention, the surface area of the silica has been calculated from the quantity of the nitrogen adsorbed by the silica at the liquid nitrogen temperature as obtained in accordance with the BET adsorption isotherm, and the average pore diameter of the silica has been obtained from the nitrogen desorption curve at the liquid nitrogen temperature in accordance with the BJH method.

The silica which is used for this invention may contain a maximum of 5% by weight of alumina, titania, zirconia, or like metal oxides, carbon or the like.

The silica having the aforementioned pore structure may be either prepared by a customary method, or obtained from a source of commercial supply. Although the silica may be used as it is, it is more desirable to use it after heat treating it at a temperature of at least $100^{\circ}C.$, preferably $100^{\circ}C$ to $500^{\circ}C$ and most preferably $120^{\circ}C$ to $500^{\circ}C$, for 0.5 to 3 hours in air. Any higher temperature should be avoided, since the preferred pore structure is likely to be destroyed.

This invention provides a solid catalyst comprising the silica having the aforementioned structure, on which particular catalytic components are supported. The catalytic components comprise at least one noble metal selected from Group VIII of the periodic table, and at least one element selected from Groups IV, V and VI of the periodic table (excluding those of the second period).

The noble metals belonging to Group VIII of the periodic table include palladium, platinum, ruthenium, rhodium, osmium and iridium. It is preferred to use palladium or platinum, and palladium is most preferred. The elements of Groups IV, V and VI of the periodic table (excluding those of the second period) mean those of Groups IV, V and VI excluding carbon, nitrogen and oxygen. Tellurium, selenium, sulfur, antimony, bismuth, germanium, tin, and lead are preferred and the use of tellurium, tin, germanium or lead is particularly preferred.

The catalyst of this invention preferably employs a combination of palladium and tellurium on the silica carrier, and more preferably, a combination of palladium and tellurium with tin, germanium or selenium. It is worthy of particular mention that the catalyst comprising a combination of palladium and tellurium with tin, germanium or selenium can maintain a very high level of activity for a very long period of time.

- 5 -

When the aforementioned noble metal and element are applied to the silica carrier, they are usually used in the form of a compound which is soluble in water or an organic solvent, e.g., an inorganic salt such as a halide, nitrate, sulfate or carbonate, or an organic salt such as an acetate, oxalate or amine salt. It is, however, equally possible to use the element per se, or an element or a compound thereof, such as an oxide, in the form of a solution in a mixture of an organic solvent and a mineral acid.

It is possible to use, for example, the compounds of the Group VIII noble metals and the Group IVA, VA and VIA elements which will hereunder be mentioned. Examples of the palladium compounds which can be used include palladium chloride, palladium sulfate, palladium nitrate and palladium oxide; examples of the platinum compounds include platinum dichloride, platinum tetrachloride, chloroplatinic acid and platinum oxide, and examples of the ruthenium compounds include ruthenium oxide, ruthenium dichloride, ruthenium tetrachloride and ruthenium hydroxide. Examples of the rhodium compounds include rhodium chloride; examples of the osmium compounds include osmium tetrachloride, and examples of the iridium compounds include iridium tetrachloride and potassium iridate. Examples of the tellurium compounds include tellurium tetrachloride and tellurium dichloride; examples of the selenium compounds include selenic acid and selenium dioxide, and examples of the antimony compounds include antimony trichloride and antimony pentachloride. Examples of the bismuth compounds include bismuth chloride and bismuth oxychloride; examples of the germanium compounds include germanium tetrachloride; examples of the tin compounds include tin dichloride; tin tetrachloride and tin acetate, and examples of the lead compounds include lead dichloride, lead chlorate and lead acetate.

The catalyst of this invention may contain 0.01 to 10% by weight, preferably 0.1 to 3% by weight, of the Group VIII noble metal based on the weight of the silica carrier, and 0.01 to 10% by weight, preferably 0.1 to 3% by weight, of the Group IV, V or VI element based on the carrier weight. The catalyst may contain 0.01 to 10 gram atoms, preferably 0.05 to 5 gram atoms of the Group IV, V or VI element per gram atom of the noble metal.

Any known method may be employed without any limitation for applying the aforementioned catalytic components to the silica carrier. For example, it is possible to form a solution of the catalytic components in a solvent such as water, an aqueous solution of hydrochloric or nitric acid or other mineral acid, and an organic solvent, immerse the carrier in that solution, and heat it to dryness. Alternatively, it is possible to immerse the carrier in a solution of the catalytic components so that the carrier may adsorb the catalytic components, and remove the remaining solution by filtration or decantation, followed by drying, or calcining after washing if required. The silica carrier may be immersed in a mixed solution containing all the necessary catalytic components in order to apply all of the components simultaneously, though it is equally satisfactory to repeat the immersion of the carrier in a plurality of solutions each containing the individual catalytic component until all the components are applied to the carrier.

The catalyst of this invention prepared as hereinabove described can advantageously maintain a high level of activity for a very long period of time when it is utilized for the manufacture of diacyloxyalkenes by reacting conjugated dienes, carboxylic acids and oxygen. When the catalyst is used for the diacyloxylation of a conjugated diene, it is preferably to treat it beforehand with a reducing agent such as hydrogen or a reducing organic compound such as hydrazine, formalin or methanol. This treatment is usually carried out at a temperature of $100^{\circ}C$ to $400^{\circ}C$.

The conjugated dienes which can be diacyloxylated in the presence of the catalyst according to this

- 7 -

invention include 1,3-butadiene, its derivatives such as isoprene, 1,3-pentadiene, 2,3-dimethyl-1,3-butadiene, 1,3-hexadiene, 2,4-hexadiene, 1,3-octadiene and 4-phenyl-1,3-butadiene, and cyclic conjugated diene compounds such as cyclopentadiene, 1,3-cyclohexadiene and 1,3-cyclooctadiene. 1,3-butadiene is particularly preferred. These conjugated dienes do not need to be pure, but may contain nitrogen, carbon dioxide, or a lower saturated hydrocarbon such as methane, ethane, propane and butane.

Any carboxylic acid, such as a saturated or unsaturated aliphatic, alicyclic or aromatic carboxylic acid, may be used for the diacyloxylation of a conjugated diene in the presence of the catalyst according to this invention. It is, however, preferred to use a lower aliphatic carboxylic acid, such as acetic acid, propionic acid or butyric acid. Acetic acid is most preferred.

Although there is no particular limitation to the conditions for the diacyloxylating reaction in the presence of the catalyst of this invention, it is suitable to employ a reaction temperature of 40°C. to 180°C., a reaction pressure ranging from atmospheric to 100 $kg/cm^2$, and a molar conjugated diene/carboxylic acid/oxygen ratio of 1:0.5 to 10:0.1 to 5. The reaction may be conducted in a batch or continuous operation in the presence of the catalyst, and the catalyst may be maintained in a suspended mode in a reaction medium, or in the form of a fixed or a fluidized bed.

The invention will now be described in further detail with reference to the following examples.

Example 1

Silica Gel ID (trade name of Fuji Davison Chemical Co., Ltd., Japan) was heated in air at 500°C for an hour to form a silica carrier having the properties shown in Table 1. 10 g of the silica carrier were immersed in a solution prepared by dissolving 0.1750 g of palladium chloride ($PdCl_2$) and 0.0536 g of tellurium dioxide ($TeO_2$) in 40 ml of 6N hydrochloric acid, and left to stand at

- 8 -

room temperature for 24 hours. Then the carrier was slowly dried by evaporation over a bath of hot water. The solid thus obtained was dried in a stream of nitrogen in a tube at 150°C. for three hours, and reduced by heating at 200°C. for three hours and 400°C. for two hours in the presence of nitrogen saturated with methanol at room temperature. There was thus prepared Catalyst A containing 1.05% by weight of palladium and 0.20% by weight of tellurium, and having a tellurium/palladium atom ratio of 0.16.

4 g of Catalyst A were placed in a stainless steel reaction tube having an inside diameter of 18 mm, and the reaction was conducted continuously at a temperature of 80°C., while the reaction tube was fed with glacial acetic acid at a rate of 12.5 ml per hour, 1,3-butadiene at a rate of 60 millimols per hour and oxygen at a rate of 40 millimols per hour. The reaction products were analyzed by gas chromatography at the end of three hours and ten hours, respectively, after the reaction was started, whereby the space time yield of the diacetoxybutene intended to be produced, and the deactivation rate constant $\alpha$ (time $^{-\frac{1}{2}}$) of the catalyst were obtained. The latter was calculated in accordance with the empirical formula showing the rate of catalyst deactivation due to coke deposition:

$$k = k_0 \exp. (- \alpha \sqrt{t})$$

wherein k: rate constant t hours after the beginning of the reaction;

$k_0$: initial rate constant; and

$\alpha$: deactivated rate constant (time $^{-\frac{1}{2}}$).

The results are shown in Table 1.

Example 2

Catalyst B containing 1.05% by weight of palladium and 0.20% by weight of tellurium, and having a tellurium/palladium atom ratio of 0.16 was prepared by repeating the procedures of Example 1, except that Silica Gel Grade-70 (tradename of Fuji Davison Chemical Co.,

Ltd., Japan) was used instead of the silica gel used in Example 1. Catalyst B was tested for the diacetoxylation of 1,3-butadiene by repeating the procedures of Example 1. The results are shown in Table 1.

As is obvious from Table 1, the use of the catalyst according to this invention permits maintenance of an excellent space time yield of diacetoxybutene for a long period of time.

### Table 1

| Example | Catalyst | Carrier Properties | | Space Time Yield (g/lit.cat./hr) | | Deactivation Rate Constant [$\alpha$] (time $-\frac{1}{2}$) |
| | | Specific surface area (m$^2$/g) | Average pore dia. (Å) | 3 hr | 10 hr | |
|---|---|---|---|---|---|---|
| 1 | A | 265 | 162 | 67 | 67 | 0.010 |
| 2 | B | 280 | 158 | 65 | 65 | 0.010 |

Comparative Examples 1 to 6

The following catalysts were prepared by using a carrier having properties which did not fall within the scope of this invention.

Catalysts C to E

Catalysts C, D and E containing the same quantities of palladium and tellurium as Catalyst A were prepared by repeating the procedures of Example 1, except that Silica Gels RD, A and B (tradenames of Fuji Davison Chemical Co., Ltd., Japan) were used respectively.

Catalyst F

Catalyst F containing the same quantities of palladium and tellurium as Catalyst A was prepared by repeating the procedures of Example 1, except that the carrier was obtained by heating Silica Gel ID (tradename of Fuji Davison Chemical Co., Ltd., Japan) at 900°C. for three hours.

Catalyst G

Catalyst G containing the same quantities of palladium and tellurium as Catalyst A was prepared by repeating the procedures of Example 1, except for the use of a carrier obtained by heating Snowtex 20 (tradename of silica of Nissan Chemical Co., Ltd., Japan, containing 20% by weight of $SiO_2$ and a maximum of 0.3% by weight of $Na_2O$, the balance being water) at 100°C. for an hour to form a gel, and heating the gel at 500°C. for an hour in the air.

Catalyst H

Catalyst H containing the same quantities of palladium and tellurium as Catalyst A was prepared by repeating the procedures of Example 1, except that the carrier was a silica gel obtained by reacting #1 water glass with nitric acid in a pH range of 9 to 10 to promote polymerization of polysilicic acid particles, and heat treating the reaction product in air at 500°C. for an hour.

The six catalysts thus obtained were tested for the diacetoxylation of 1,3-butadiene by repeating the procedures of Example 1. The space time yield of diacetoxybutene, and the rate constant of catalyst deactivation based on the empirical formula indicating catalyst deactivation due to coke deposition were obtained at the end of three hours after the beginning of the reaction. The results are shown in Table 2, and teach that in order to obtain a catalyst maintaining a high level of activity for a long period of time, it is necessary to use a carrier composed of silica having a specific surface area of at least 200 $m^2$/g and an average pore diameter of at least 100 Å.

0036715

- 11 -

### Table 2

| Comparative Example | Catalyst | Carrier Properties | | Space Time Yield (g/lit.cat./hr) | Deactivation Rate Constant [$\alpha$] (time-$\frac{1}{2}$) |
|---|---|---|---|---|---|
| | | Specific surface area $m^2/g$ | Average Pore dia. $\overset{\circ}{A}$ | | |
| 1 | C | 789 | 22 | 15 | 0.250 |
| 2 | D | 650 | 22 | 5 | 0.250 |
| 3 | E | 517 | 58 | 34 | 0.042 |
| 4 | F | 187 | 184 | 33 | 0.010 |
| 5 | G | 208 | 74 | 38 | 0.014 |
| 6 | H | 155 | 132 | 22 | 0.010 |

### Examples 3 and 4

4 g of Catalyst A prepared in Example 1 were placed in a stainless steel reaction tube having an inside diameter of 14 mm, and the reaction was conducted continuously at a temperature of 80°C. and 95°C. and a pressure of 40 kg/cm$^2$ for 500 hours, while the reaction tube was fed with glacial acetic acid at a rate of 12.5 ml per hour, liquid 1,3-butadiene at a rate of 5.2 ml per hour and nitrogen containing 6% by volume of oxygen at a rate of 270 ml per minute (standard condition). Table 3 shows the initial space time yield of diacetoxybutene, the selectivity of the catalyst for 1,4-diacetoxybutene-2, and the half life of the catalyst obtained in accordance with the empirical formula indicating catalyst deactivation due to coke deposition. As is obvious from the results shown in Table 3, the catalyst of this invention shows a very high space time yield and selectivity for 1,4-diacetoxybutene, and maintains its high activity for a very long period of time.

### Table 3

| Example | Reaction temp. (°C) | Space Time Yield (g/lit.cat./hr) | 1,4-Selectivity (%) | Half Life (days) |
|---|---|---|---|---|
| 3 | 80 | 222 | 92 | 638 |
| 4 | 95 | 313 | 91 | 268 |

### Comparative Example 7

The diacetoxylation of 1,3-butadiene was performed by repeating the procedures of Example 3, except that Catalyst E according to Comparative Example 3 was used. The catalyst showed a space time yield of 109 g/lit.cat.hr, a selectivity for 1,4-diacetoxybutene-2 of 92%, and a half life of 25 days.

Comparative Example 8

A catalyst containing the same quantities of palladium and tellurium as Catalyst A was prepared by repeating the procedures of Example 1, except for the use as a carrier of coconut shell activated carbon having a specific surface area of 892 $m^2$/g, and an average pore diameter of 24 Å. The catalyst thus obtained was tested for the diacetoxylation of 1,3-butadiene by repeating the procedures of Example 1. The catalyst showed a space time yield of 69 g/lit.cat./hr with the lapse of three hours after the reaction was started, but it sharply decreased to 55 with the lapse of ten hours [deactivation rate constant = 0.072 (time $\frac{-1}{2}$)].

The same catalyst was also tested for the diacetoxylation of 1,3-butadiene at a temperature of 100°C. by repeating otherwise the procedures of Example 3. It showed an initial space time yield of 215 g/lit.cat./hr., a selectivity for 1,4-diacetoxybutene-2 of 92%, and a half life of 17 days. The catalyst was also tested for the diacetoxylation of 1,3-butadiene at a reaction temperature of 100°C. by introducing elemental sulfur into the reaction system for the purpose of maintaining the activity of the catalyst, and otherwise repeating the procedures of Example 3. The catalyst showed a space time yield of 209 g/lit. cat./hr., and a selectivity for 1,4-diacetoxybutene-2 of 92%, but its half life showed an increase only to 42 days.

Examples 5 to 7

Catalysts I, J and K were prepared by repeating the procedures of Example 1, except that the solution in which the silica carrier was immersed was a solution prepared by dissolving 0.1750 g of palladium chloride ($PdCl_2$),

0.0536 g of tellurium dioxide ($TeO_2$), and 0.1928 g of tin tetrachloride ($SnCl_4$), or 0.1593 g of germanium tetrachloride ($GeCl_4$), or 0.2453 g of lead nitrate [$Pb(NO_3)_2$] in 40 ml of 6N hydrochloric acid. These catalysts contained 1.05% by weight of palladium, 0.02% by weight of tellurium, and 0.88% by weight of tin (Catalyst I), 0.54% by weight of germanium (Catalyst J), or 1.53% by weight of lead (Catalyst K), and had a tellurium/palladium atom ratio of 0.16, and a tin, germanium or lead/palladium atom ratio of 0.75.

These catalysts were tested for the diacetoxylation of 1,3-butadiene in accordance with the procedures of Example 1. At the end of three hours after the reaction was started, the space time yield of diacetoxybutene and the rate constant for catalytic deactivation were determined as shown in Table 4.

Comparative Examples 9 to 11

Catalysts L, M and N were prepared by repeating the procedures of Examples 5 to 7, except that the carrier used in Comparative Example 3 for the preparation of Catalyst E was employed. The three catalysts thus obtained were tested for the diacetoxylation of 1,3-butadiene in accordance with the procedures set forth in Example 1. The results are shown in Table 4.

As is obvious from the results shown in Table 4, the catalysts composed of the silica carrier according to this invention, and the three catalytic metal components according to Examples 5 to 7 can maintain an extremely higher level of activity for a long period of time.

0036715

Table 4

| | Catalyst | Catalytic Components | Space Time Yield (g/lit. cat./hr) | Deactivation Rate Constant $[\alpha]$ (time$-\frac{1}{2}$) |
|---|---|---|---|---|
| Example 5 | I | Pd-Te-Sn | 110 | 0.010 |
| 6 | J | Pd-Te-Ge | 105 | 0.010 |
| 7 | K | Pd-Te-Pb | 100 | 0.010 |
| Comparative Example 9 | L | Pd-Te-Sn | 51 | 0.040 |
| 10 | M | Pd-Te-Ge | 46 | 0.046 |
| 11 | N | Pd-Te-Pb | 40 | 0.045 |

0036715

WHAT WE CLAIM IS:

1. A catalyst for diacyloxylation of conjugated dienes, including a carrier supporting thereon at least one noble metal belonging to Group VIII of the periodic table, and at least one element belonging to Groups IV, V or VI of the periodic table (but excluding those of the second period), characterized in that the carrier comprises silica having a specific surface area of at least 200 $m^2$/g, and an average pore diameter of at least 100 $\overset{o}{A}$.

2. A catalyst according to Claim 1 in which the silica has a specific surface area of 250 to 1,000 $m^2$/g and an average pore diameter of 100 to 500 $\overset{o}{A}$.

3. A catalyst according to Claim 1 or Claim 2, in which the silica has been heat treated at a temperature in the range of 100° to 500°C in air before application thereto of the noble metal and elements.

4. A catalyst according to any of Claims 1 to 3, in which the or each element is selected from Groups IVA, VA, and VIA.

5. A catalyst according to Claim 4 in which the or each element is selected from tellurium, selenium, sulfur, antimony, bismuth, germanium, tin and lead.

6. A catalyst according to Claim 5 in which the or each element is selected from tellurium, tin, germanium and lead and the Group VIII noble metal is palladium.

7. A catalyst according to any of Claims 1 to 3, which comprises tellurium and palladium.

0036715

8. A catalyst according to any of Claims 4 to 7, which comprises 0.01 to 10% by weight of the Group VIII noble metal and 0.01 to 10% by weight of the Group IVA, VA, or VIA element.

9. The catalyst according to any of Claims 1 to 8, which has been treated before use with a reducing agent at a temperature in the range of $100^\circ$ to $400^\circ$C.

10. The catalyst according to Claim 9 in which the reducing agent is methanol.

European Patent Office

**EUROPEAN SEARCH REPORT**

Application number

EP 81 30 0860

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | FR - A - 2 121 693 (HOECHST) <br> * Page 1, lines 1-28; page 2, lines 4-17; claims * <br> & US - A - 3 939 199 | 1,2 | B 01 J 35/10 |
| | FR - A - 2 071 942 (B.P.) <br> * Pages 1,2,3,8; page 22, example 9, claims 1-5 * <br> & US - A - 3 726 809 | 1,2,9, 10 | |
| | FR - A - 2 015 130 (NAT. DISTILLERS AND CHEM. CORP.) <br> * Pages 1,2,3; claims 1-15 * <br> & US - A - 3 794 713 | 1,2,3 | TECHNICAL FIELDS SEARCHED (Int. Cl.³) <br><br> B 01 J 35/10 <br> C 07 C 67/055 <br> 69/16 |
| | US - A - 3 755 423 (T. ONODA) <br> * Abstract, column 2, lines 1-29; column 3, line 16-63; column 4, lines 13-25; column 5,6; examples 1,9; claims 1-29 * | 1,4,5, 6,7,8, 9,10 | |
| | FR - A - 2 120 147 (TOYO SODA) <br> * Page 3, line 22 - page 4, line 22; claims 1-12 * | 1,4,5, 6,8,9 | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| A | US - A - 4 102 779 (HENSLEY JR.) | | |
| A | US - A - 4 053 565 (KREKELER et al.) | | |
| | The present search report has been drawn up for all claims | | &: member of the same patent family, corresponding document |

| Place of search | Date of completion of the search | Examiner |
|---|---|---|
| The Hague | 30-06-1981 | LO CONTE |

EPO Form 1503.1 06.78